# EUROPEAN PATENT APPLICATION

(11) **EP 4 445 828 A1**
(43) Date of publication of application: **16.10.2024**
(21) Application number: 22904514.1
(22) Date of filing: 17.11.2022
(51) Int. Cl.: A61B 3/117, A61B 3/00, A61B 3/14, A61B 5/00, A61B 3/12

(54) **AUTOFLUORESCENCE IMAGING DEVICE AND OPERATION METHOD THEREOF, AUTOFLUORESCENCE IMAGE EVALUATION DEVICE AND EVALUATION METHOD THEREOF**

(30) Priority: 07.12.2021 KR 20210173813; 02.03.2022 KR 20220026607
(71) Applicant: Korea University Research and Business Foundation, Seoul 02841 (KR)
(72) Inventor: EOM, Young Sub, Seoul 05502 (KR); KIM, Seong Woo, Seoul 06544 (KR); SUH, Young Woo, Seoul 06267 (KR)
(74) Representative: IPAZ
(86) International application number: PCT/KR2022/018233
(87) International publication number: WO 2023/106681

(57) **Abstract**

An autofluorescence imaging device, according to one embodiment of the present disclosure, comprises: a light source for illuminating an examined eye through a preset optical path; an image sensor for imaging the examined eye of which at least a portion is emitting autofluorescence by the illumination from the light source; and a filter disposed between the image sensor and the examined eye, wherein the filter may comprise a plurality of portions each having mutually different light transmittances.

## Description

### TECHNICAL FIELD

The present disclosure relates to an autofluorescence (AF) imaging device of an examined eye and an operation method thereof and an autofluorescence image evaluation device and an evaluation method thereof, and more specifically, to AF imaging and evaluation based on a filter capable of quantitatively evaluating a degree of progress of ocular disease or presbyopia.

### BACKGROUND ART

Conventionally, there was a lens opacities classification system (LOCS) as a standard for classifying nuclear cataracts. The LOCS divides a crystalline lens into three parts: cortex, nucleus, and posterior capsule (posterior lens capsule), and the cortex and the posterior capsule are divided into five stages according to opacity, and the nucleus is divided into six stages according to opacity and tone. A medical staff decides the nuclear cataract based on the LOCS as a result of directly observing a monolayer of the crystalline lens with an eye using a slit lamp microscope. Cataract removal surgery is performed by a method of removing an opaque crystalline lens and inserting an artificial lens.

However, there is a case where the cataract removal surgery is not required depending on a degree of progress of cataract, but the degree of progress of cataract is currently decided only through direct observation by the medical staff, such that there is a problem that a decision result may be different per medical staff, and there is a possibility that a patient who does not need surgery will undergo the cataract removal surgery. Accordingly, a method capable of quantitatively deciding a degree of progress of cataract or a degree of progress of presbyopia is required.

In addition, conventionally, there is a fundus autofluorescence (FAF) imaging technology. Unlike fluorescein angiography, FAF imaging does not need to inject a fluorescent dye in order to image the retina, and generates an image by utilizing fluorescent properties of lipofuscin within the retinal pigment epithelium (RPE). Since an abnormal pattern of autofluorescence (AF) in an FAF image acts as a marker for retinal disease, conventional FAF was used to evaluate retinal disease or abnormalities and was not used to evaluate crystalline lens abnormalities.

Korea Patent Publication No. 10-1643953 is a technology that determines an intensity histogram from an FAF image of a patient and compares the intensity histogram with a control group to determine abnormalities, but Korea Patent Publication No. 10-1643953 is also a technology that determines retinal disease and it is difficult to use Korea Patent Publication No. 10-1643953 for quantitative evaluation of crystalline lens abnormalities including the degree of progress of presbyopia, the degree of progress of cataract, or the like.

### DISCLOSURE OF INVENTION

### Technical problem

An embodiment of the present disclosure provides an autofluorescence imaging device of an examined eye based on a filter for evaluating crystalline lens abnormalities, and an operation method thereof.

Another embodiment of the present disclosure provides a device and a method of analyzing and evaluating an autofluorescence image of an examined eye captured based on a filter for evaluating crystalline lens abnormalities.

Another embodiment of the present disclosure provides a filter that may be used in an autofluorescence imaging device of an examined eye in order to evaluate crystalline lens abnormalities.

### Solution to problem

An embodiment of the present disclosure provides an autofluorescence imaging device of an examined eye and an operation method thereof.

According to an embodiment of the present disclosure, for an autofluorescence imaging device and an operation method thereof, the autofluorescence image of an examined eye is quantitatively evaluated.

According to an embodiment of the present disclosure, an autofluorescence imaging device includes: a light source illuminating an examined eye through a preset optical path; an image sensor imaging the examined eye of which at least a portion emits light as autofluorescence by the illumination of the light source; and a filter disposed between the image sensor and the examined eye, in which the filter includes a plurality of portions each having different light transmittances.

According to an embodiment of the present disclosure, an autofluorescence image evaluation device includes: a processor; and a memory electrically connected to the processor and storing at least one code executed by the processor, in which the memory stores a code causing the processor to analyze an autofluorescence image of an examined eye and determine information related to an opacity degree or a cataract grade of the examined eye, and the autofluorescence image is an image generated based on output of an image sensor on which autofluorescence of the examined eye generated by illumination of a light source passes through a filter disposed between the image sensor and the examined eye and including a plurality of portions each having different light transmittances and is then incident.

According to an embodiment of the present disclosure, an operation method of an autofluorescence imaging device includes: emitting light from a light source so as to illuminate an examined eye through a preset optical path, by a processor; and imaging the examined eye of which at least a portion emits light as autofluorescence by the illumination of the light source by controlling an image sensor, by the processor, in which the imaging of the examined eye includes generating an output signal by the image sensor based on the autofluorescence of the examined eye passing through a filter including a plurality of portions each having different light transmittances and then incident on the image sensor.

According to an embodiment of the present disclosure, an evaluation method of an autofluorescence image evaluation device includes: receiving at least a portion of an autofluorescence image of an examined eye by a processor; and analyzing the autofluorescence image and determining information related to an opacity degree or a cataract grade of the examined eye, by the processor, in which the autofluorescence image is an image generated based on output of an image sensor on which autofluorescence of the examined eye generated by illumination of a light source passes through a filter disposed between the image sensor and the examined eye and including a plurality of portions each having different light transmittances and is then incident.

### Advantageous effects of the invention

According to an embodiment of the present disclosure, an autofluorescence imaging device and an operation method thereof may capture an autofluorescence image capable of quantitatively deciding crystalline lens abnormalities.

according to an embodiment of the present disclosure, an autofluorescence image evaluation device and an evaluation method thereof may quantitatively evaluate crystalline lens abnormalities including a degree of progress of presbyopia, a degree of progress of cataract, or the like, based on an autofluorescence image.

Embodiments of the present disclosure may reduce inconvenience of patients and reduce an unnecessary surgery cost, by quantitatively evaluating crystalline lens abnormalities of the patients and preventing unnecessary cataract surgery.

### BRIEF DESCRIPTION OF DRAWINGS

FIG. 1 is a diagram illustrating an environment in which an autofluorescence imaging device according to an embodiment of the present disclosure captures an image using a filter for evaluating crystalline lens abnormalities.
FIG. 2 is a schematic block diagram illustrating a configuration of the autofluorescence imaging device according to an embodiment of the present disclosure.
FIGS. 3 and 4 are flowcharts for describing an operation method of the autofluorescence imaging device according to an embodiment of the present disclosure.
FIGS. 5 and 6 are diagrams illustrating embodiments of filters for evaluating crystalline lens abnormalities according to an embodiment of the present disclosure.
FIG. 7 is a schematic block diagram illustrating a configuration of an autofluorescence image evaluation device according to an embodiment of the present disclosure.
FIG. 8 is a flowchart for describing an evaluation method of the autofluorescence image evaluation device according to an embodiment of the disclosure.
FIGS. 9 and 10 are diagrams for describing an evaluation method of the autofluorescence image evaluation device according to an embodiment of the present disclosure.

### BEST MODE FOR CARRY OUT THE INVENTION

Hereafter, embodiments of the present disclosure will be described in detail with reference to the accompanying drawings and the same or similar components are given the same reference numerals regardless of the numbers of figures and are not repeatedly described. In addition, terms "module" and "unit" for components used in the following description are used only to easily make the disclosure. Therefore, these terms do not have meanings or roles that distinguish from each other in themselves. Further, when it is decided that a detailed description for the known art related to the present disclosure may obscure the gist of the present disclosure, the detailed description will be omitted. Further, it should be understood that the accompanying drawings are provided only in order to allow exemplary embodiments of the present disclosure to be easily understood, and the spirit of the present disclosure is not limited by the accompanying drawings, but includes all the modifications, equivalents, and substitutions included in the spirit and the scope of the present disclosure.

Terms including ordinal numbers such as "first," "second," etc., may be used to describe various components, but the components are not to be construed as being limited to the terms. The terms are used only to distinguish one component from another component.

It is to be understood that when one element is referred to as being "connected to" or "coupled to" another element, it may be connected directly to or coupled directly to another element or be connected to or coupled to another element, having the other element intervening therebetween. On the other hand, it should be understood that when one element is referred to as being "connected directly to" or "coupled directly to" another element, it may be connected to or coupled to another element without the other element interposed therebetween.

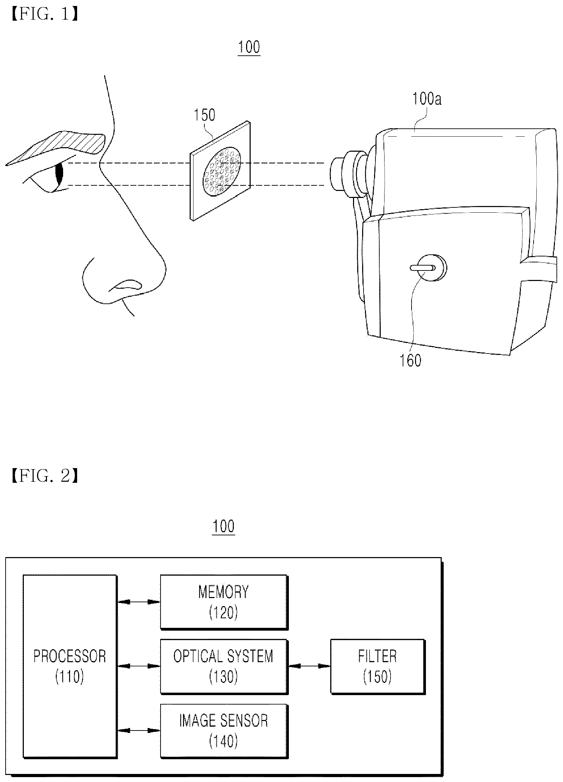

An environment and a configuration for driving an autofluorescence imaging device according to an embodiment of the present disclosure will be described with reference to FIGS. 1 and 2.

An autofluorescence imaging device 100 according to an embodiment of the present disclosure may include a filter (hereinafter referred to as an 'analysis filter') 150 set to be disposed inside or outside a body 100a including a camera and provided for evaluating crystalline lens abnormalities. In addition, in another embodiment, the analysis filter 150 may be implemented in the form of glasses, and an autofluorescence image may be captured in a state in which a subject wears the analysis filter 150 having the form of glasses when the autofluorescence imaging device 100 captures the autofluorescence image.

The autofluorescence imaging device 100 may be set to illuminate an examined eye of a patient by allowing a light source generating excitation light to emit the excitation light and guiding the excitation light so that the excitation light passes through a preset optical path.

The autofluorescence imaging device 100 may include an image sensor 140 on which autofluorescence (AF) emitted as autofluorescence of the examined eye by the excitation light is incident, and the analysis filter may be disposed between the image sensor 140 and the examined eye. The analysis filter may be set to be disposed inside or outside the body 100a. An embodiment in which the analysis filter is disposed outside the body 100a includes an embodiment in which the subject wears the analysis filter 150 having the form of glasses. That is, the autofluorescence generated in the examined eye by the excitation light may pass through the analysis filter 150 and be then incident on the image sensor 140.

The autofluorescence may be generated in a crystalline lens as well as lipofuscin in the retinal pigment epithelium (RPE) of the examined eye.

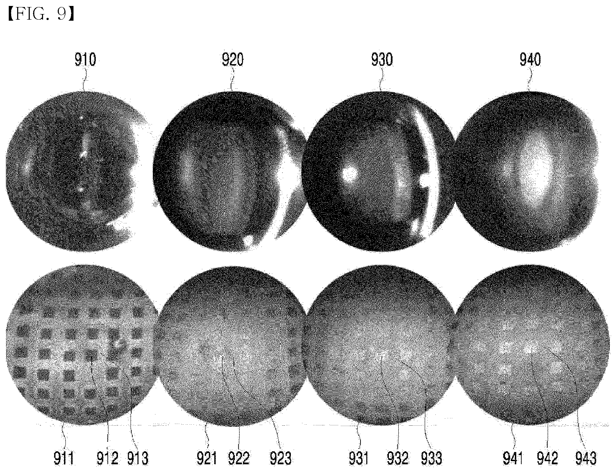

That is, when autofluorescence images of crystalline lenses having different opacity degrees as illustrated in FIG. 9 are captured based on the analysis filter according to an embodiment of the present disclosure, the present inventors have confirmed that brightness of portions of the images was increased due to autofluorescence of the crystalline lenses unlike general expectation that autofluorescence of the retinas would be blocked by opacity of the crystalline lenses, and as a result, it is possible to quantitatively decide crystalline lens abnormalities based on a change in gray level of the autofluorescence image corresponding to a figure pattern of the analysis filter according to an embodiment of the present disclosure. This is decided to be a complex phenomenon due to a portion of the autofluorescence of the retina obscured by opacity, a portion of the autofluorescence of the retina incident on the image sensor 140 by scattering, refraction, or the like, and scattering of the autofluorescence of the crystalline lens. Accordingly, a degree of progress of cataract or a degree of progress of presbyopia related to the opacity of the crystalline lens may be quantitatively evaluated based on the analysis filter according to an embodiment of the present disclosure.

The camera of the autofluorescence imaging device 100 may include the image sensor (a charge coupled device (CCD) or a complementary metal oxide semiconductor (CMOS)) 140, and may adjust a field of view and a focus so as to be capable of imaging autofluorescence from the examined eye.

The autofluorescence imaging device may include an optical system 130 including a mirror, a lens, and the like, so that the light emitted from the light source to the examined eye passes through a first filter set to pass only light of a specific wavelength therethrough and is then guided to the examined eye.

The first filter may be selected to reduce a wavelength that does not correspond to a wavelength band (e.g., about 470 nm or a wavelength near 470 nm) exciting a specific cell in order to generate autofluorescence. However, embodiments of the present disclosure are not limited to a specific band of an excitation wavelength, and other wavelength bands of excitation light induced in the examined eye according to other configurations of the autofluorescence imaging device are also possible. When the light source is set to emit only light of a specific band, the first filter may be omitted.

The autofluorescence generated in the examined eye by excitation light may pass through the lens, the mirror, and the like, of the optical system 130 and be then incident on the image sensor 140 of the camera. In an embodiment, the autofluorescence imaging device 100 may include a second filter for removing light of an unwanted band or light that is not the autofluorescence, in the autofluorescence.

The analysis filter 150 may allow the autofluorescence generated in the examined eye to pass therethrough before being incident on the image sensor 140 and may be set to be disposed before or after the second filter.

When the analysis filter 150 is disposed outside the body 100a, a structure that may be attached to a holder holding the forehead and the chin of the patient may be included or a structure (e.g., a wheel mounted with the analysis filter 150 and a wheel driving motor) that may be rotatably disposed in front of the camera so that the analysis filter 150 may be selectively used depending on an imaging mode may be included.

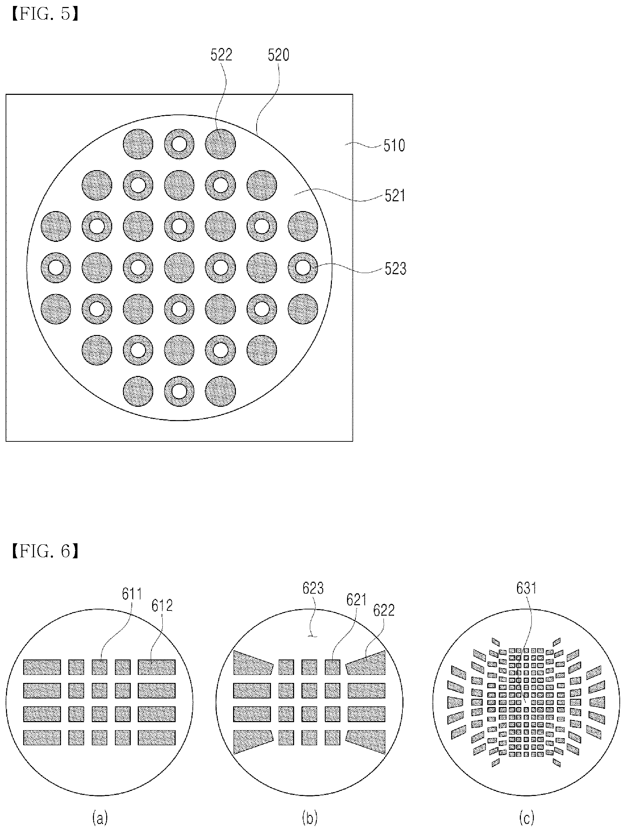

The analysis filter 150 may include a plurality of parts each having different light transmittances, and will be described in detail below with reference to FIGS. 5 and 6.

The autofluorescence imaging device 100 may store the autofluorescence image generated based on output of the image sensor 140 in a memory 120 or transmit the autofluorescence image to an external device connected through a communication module. The autofluorescence imaging device 100 may include a processor 110 performing post-processing of a wavelength and brightness of the light source or the generated autofluorescence image. In another embodiment, the autofluorescence imaging device 100 may be connected to an external control computing device, and the control computing device may perform setting of the autofluorescence imaging device 100 or post-processing of the captured autofluorescence image. In the present specification, the processor 110 of the autofluorescence imaging device 100 may be understood as a concept including a computing device implemented separately from the body 100a.

The control computing device may include, for example, a tablet computer, a personal computer (PC), a laptop computer, a smartphones, and the like.

The communication module may include components similar to some or all of components of a communication unit 210 of an autofluorescence image evaluation device 200 to be described later, and the body 100a may be connected to the control computing device in various manners such as a cable, a local area network (LAN), wireless-fidelity (Wi-Fi), and short-range wireless communication.

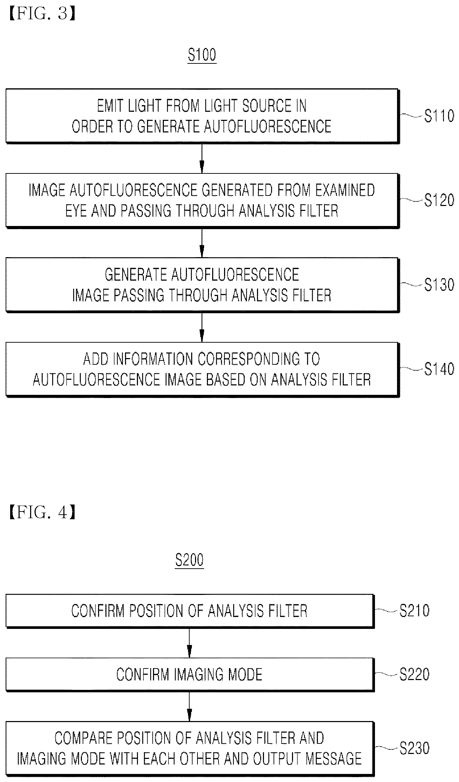

An operation method of the autofluorescence imaging device 100 according to an embodiment of the present disclosure will be described with reference to FIGS. 2 and 3.

The autofluorescence imaging device 100 controls the light source to emit light including a wavelength band in which the autofluorescence of the examined eye may be generated (S 1 10).

The light generated from the light source may include a wavelength band (e.g., about 470 nm or a wavelength near 470 nm) exciting a specific cell in order to generate the autofluorescence or may include only that wavelength band.

The light emitted from the light source may be guided to pass through the optical system 130 such as an optical filter, the mirror, or the lens and then illuminate the examined eye. The processor 110 may control angles, positions, and the like, of components of the optical system 130.

The light emitted from the light source and guided to the examined eye through the optical system 130 generates autofluorescence in a retina cell, a crystalline lens, or the like, of the examined eye, and the generated autofluorescence passes through the analysis filter 150 and is then incident on the image sensor 140 (S120).

In an embodiment, the autofluorescence may pass through the optical system 130, such as the mirror, the optical filter, or the lens before or after passing through the analysis filter 150.

The image sensor 140 outputs an electrical signal based on the incident autofluorescence, and the processor 110 generates an autofluorescence image based on the output of the image sensor 140 (S130).

In an embodiment, the autofluorescence imaging device 100 may include information meaning a type of image for distinguishing the autofluorescence image generated based on the analysis filter 150 according to an embodiment of the present disclosure from a conventional general autofluorescence image in header information or the like of the autofluorescence image (S140). Alternatively, the autofluorescence imaging device 100 may generate a user definition message meaning that the autofluorescence imaging device 100 based on the analysis filter 150 according to an embodiment of the present disclosure operates as an SCU and transmits the image to a picture archiving and communication system (PACS) server device based on digital imaging and communications in medicine (DICOM).

The autofluorescence imaging device 100 may store the autofluorescence image generated based on the analysis filter 150 according to an embodiment of the present disclosure in the control computing device or transmit the autofluorescence image to a clinical information system (CIS), PACS, or hospital information system (HIS) server device.

In an embodiment, the autofluorescence imaging device 100 may confirm a position of the analysis filter 150 (S210), confirm an imaging mode of the fluorescence imaging device 100 (S220), and then output a warning message or a confirmation message through a display, a sound, a warning light, or the like, based on a comparison result between the position of the analysis filter 150 and the imaging mode (S230), in order to capture an autofluorescence image based on the analysis filter 150 according to an embodiment of the present disclosure.

When the analysis filter 150 is rotated and positioned in a rotary manner by the wheel, the autofluorescence imaging device 100 may decide the position of the analysis filter 150 (and whether the analysis filter 150 is attached or detached) by a sensor deciding the position of the analysis filter 150 according to the rotation and a sensor deciding whether or not the analysis filter 150 is coupled to the holder when the analysis filter 150 is coupled to the holder in a detachable structure. Alternatively, the autofluorescence imaging device 100 may decide the position of the analysis filter 150 (and whether the analysis filter 150 is attached or detached) by recognizing output of a radio frequency (RF) chip mounted on the analysis filter 150. When the analysis filter 150 is implemented in the form of glasses, it may be decided whether or not the subject has worn the analysis filter 150 based on a human body sensing sensor (infrared ray, etc.).

The autofluorescence imaging device 100 may be implemented to be drivable in a conventional autofluorescence imaging mode in addition to an autofluorescence imaging mode based on the analysis filter 150 according to an embodiment of the present disclosure. In this case, the autofluorescence imaging device 100 may include a mechanical interface (e.g., a rotary lever 160 of FIG. 1), a display of the body 150a, or an electrical interface of the control computing device for selecting an imaging mode. Accordingly, the autofluorescence imaging device 100 may confirm whether or not the imaging mode is the autofluorescence imaging mode based on the analysis filter 150 according to an embodiment of the present disclosure and then output a warning message when the analysis filter 150 is at an imaging position even though the imaging mode is set to the conventional autofluorescence imaging mode or vice versa. Alternatively, the autofluorescence imaging device 100 may output a message notifying that the analysis filter 150 is activated when the analysis filter 150 is disposed at the imaging position regardless of the imaging mode.

Embodiments of the analysis filter 150 according to an embodiment of the present disclosure will be described with reference to FIGS. 5 and 6.

The analysis filter 150 may include an outer peripheral portion 510 that may be attached to a holder holding the forehead and the chin of the patient or coupled to the body 100a and a filter portion 520 through which the autofluorescence generated in the examined eye passes. The filter portion 520 may include a plurality of portions 521, 522, and 523 having different light transmittances. Light transmittance in the present specification is a concept including that luminous intensity or other characteristics of light change due to scattering and refraction after the passage of light.

For example, the filter portion 520 may include a first part 521 that is transparent and second parts 522 and 523 where a plurality of figures having lower light transmittance than the first part 521 are formed.

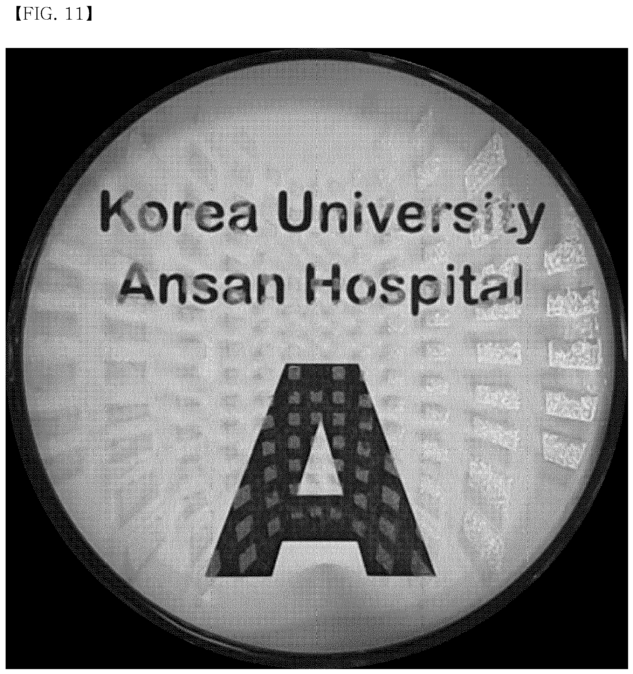

The first part 521 may be transparent or opaque, but may have higher light transmittance than the second parts 522 and 523. The filter portion 520 may be made of glass or plastic, and may be implemented in a form in which a film having a different color or material is attach to the second parts 522 and 523 or surface roughness of the second parts 522 and 523 is changed. When the filter portion 520 is made of the glass, the second parts 522 and 523 may be frosted glass due to friction or corrosion. When the analysis filter 150 is implemented with glass, the second parts 522 and 523 may be implemented with a sand blast that performs pneumatic spraying of etched glass using a glass etchant or laser processing, sand, or emery. FIG. 11 is a photograph taken after the analysis filter 150, which is an embodiment of the present disclosure, is implemented in the form of FIG. 6C by a laser processing method and positioned at an upper end of a printed matter. It can be seen from FIG. 11 that light transmittance of the second part is lower than light transmittance of the first part.

In an embodiment, the second part 522 of the analysis filter 150 may be implemented as a plurality of figure patterns, and may be a plurality of ring-shaped figures and a plurality of circle-shaped figures. In the case of the ring-shaped figure, a circular inner portion inside a ring may be the first part. As described in detail below, the autofluorescence image evaluation device 200 may evaluate crystalline lens abnormalities based on a result of measuring gray levels in portions of an autofluorescence image corresponding to the first part 521 and/or the second part 522 and 523 of the analysis filter. In the case of the ring-shaped figure, the autofluorescence image may be analyzed with a gray level of the circular inner portion inside the ring and a gray level of a ring portion as the first part and the second part, respectively. Accordingly, in the case of the ring-shaped figure, a position for analyzing the gray level may be easily specified through figure recognition.

In an embodiment, a plurality of figure patterns having low light transmittance in the analysis filter 150 may be disposed to be spaced apart from each other, and a plurality of figures may be disposed in a vertically symmetrical or horizontal symmetrical form in the filter portion.

Various embodiments of the analysis filter 150 will be described with reference to FIG. 6. FIG. 6 illustrates embodiments in which the outer peripheral portion of the analysis filter 150 is omitted and only the filter portion of the analysis filter 150 is illustrated.

Referring to FIG. 6A, second parts 611 and 612 of the analysis filter 150 may be a plurality of quadrangular figures of which inner portions have uniform light transmittance and which have lower light transmittance than other part (first part). In addition, an area of a figure of the second part 612 far from the center (positioned in a peripheral portion or close to the outer peripheral portion) of the filter portion may be greater than that of a figure of the second part 611 close to the center of the filter portion.

In an embodiment, a horizontal ratio of the figure of the second part 612 far from the center may be greater. In this case, in the case of the autofluorescence imaging device that scans the examined eye, portions of an image corresponding to the second part in the autofluorescence image may be displayed in the image at the same size so as to be appropriate for barrel distortion due to a form of an eyeball in which a horizontal ratio is greater, a spherical shape of the eyeball, or a form of a lens.

Referring to FIG. 6B, a figure of a second part 622 far from the center may be a quadrangle having a different shape from a figure 621 of a second part closer to the center. For example, the figure 621 of the second part may be a square or a rectangle, and the figure of the second part 622 may be a parallelogram or a trapezoid. In this case, a result that more actively reflects barrel distortion may be confirmed.

Referring to FIGS. 6A to 6C, in an embodiment, in the case of a plurality of figures corresponding to the second part of the filter portion, the numbers of figures distributed along a horizontal line and a vertical line crossing the center of the filter portion may be different from each other. For example, the number of figures distributed along the vertical line may be smaller. That is, in the distribution of the figures along the vertical line, the figure of the second part may not be disposed in a part 623 close to the outer peripheral portion. It may be a figure pattern that takes into account a case where the eyeball has an earth ellipse shape of which a horizontal ratio is greater or a case where left and right parts of the examined eye are more imaged due to the eyelid or the like

Referring to FIG. 6C, a plurality of figures corresponding to the second part of the filter portion may be disposed in a radial form from the center point.

Referring to FIG. 6C, the plurality of figures corresponding to the second part of the filter portion may not be disposed near a center portion 631. When the autofluorescence image is captured using the analysis filter 150, a point for guiding a sight line of the patient is required. Accordingly, by not disposing the plurality of figures corresponding to the second part near the central portion 631, it is possible to guide the sight line of the patient and capture an appropriate autofluorescence image. In an embodiment, a part where the figures corresponding to the second part are not disposed may be a position of the central portion 631 or a position spaced apart from the central portion 631 by a predetermined position in a downward direction or an upward direction. Due to a structure of an eyeball of a person, even though the person intensively views the center, an image may be captured differently in a fundus autofluorescent photograph. Therefore, in order to reflect this, the figures corresponding to the second part may not be disposed at the position spaced apart from the central portion 631 of a pattern by the predetermined position in the downward direction or the upward direction.

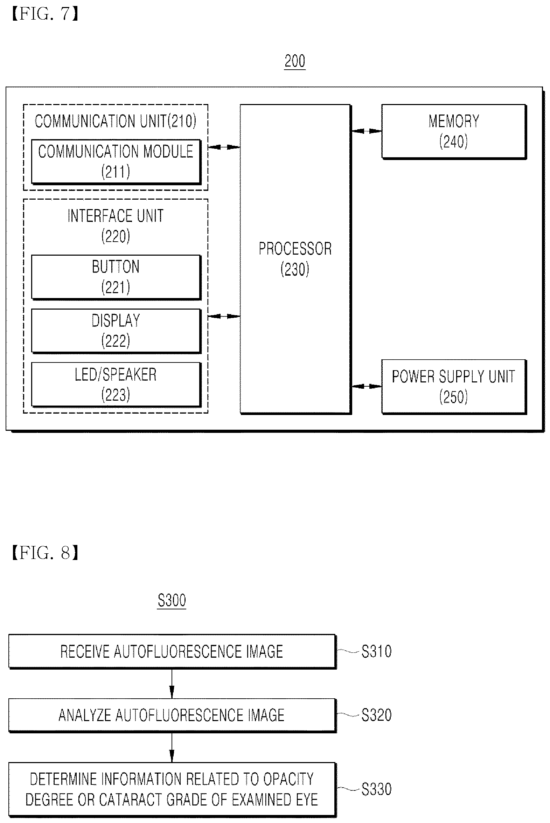

A configuration of an autofluorescence image evaluation device 200 according to an embodiment of the present disclosure will be described with reference to FIG. 7.

The autofluorescence image evaluation device 200 may analyze the autofluorescence image generated based on the analysis filter in the autofluorescence imaging device and quantitatively evaluate crystalline lens abnormalities of the examined eye.

The autofluorescence image evaluation device 200 may be a control computing device that controls the autofluorescence imaging device or a CIS/PACS/HIS server device, or may be implemented as a standalone computing device. The autofluorescence image evaluation device 200 may be a computing device capable of loading and analyzing the autofluorescence image, such as a tablet computer, a laptop computer, a PC, or a smartphone.

The autofluorescence image evaluation device 200 may include a communication unit 210 that receives the autofluorescence image from the CIS/PACS/HIS server device, the autofluorescence imaging device, or the control computing device of the autofluorescence imaging device.

The communication unit 210 may include a wireless communication unit or a wired communication unit.

The wireless communication unit may include at least one of a mobile communication module, a wireless Internet module, a short-range communication module, and a position information module.

The mobile communication module transmits and receives a wireless signal to and from at least one of a base station, an external terminal, and a server on a mobile communication network built according to long term evolution (LTE), which is a communication method for mobile communication.

The wireless Internet module is a module for accessing wireless Internet, may be mounted inside or outside the autofluorescence image evaluation device 200, and may use wireless LAN (WLAN), Wi-Fi, Wi-Fi direct, digital living network alliance (DLNA), or the like.

The short-range communication module is a module for transmitting and receiving data through short-range communication, and may use Bluetooth^{™}, radio frequency identification (RFID), infrared data association (IrDA), ultra wideband (UWB), ZigBee, near field communication (NFC), or the like.

The position information module is a module for obtaining a position of the autofluorescence image evaluation device 200, and may be a global positioning system (GPS) module based on satellite navigation technology or a module obtaining a position based on wireless communication with a wireless communication base station or a wireless access point. The position information module may include a WiFi module.

In an embodiment, the autofluorescence image evaluation device 200 may include an interface unit 220 for user input, and the interface unit 220 may include an input unit or an output unit.

The input unit may include a user interface (UI) including a microphone and a touch interface 221 for receiving information from a user, the user interface may include not only a mouse and a keyboard, but also mechanical and electronic interfaces implemented in the autofluorescence image evaluation device, and a manner and a form of the user interface are not particularly limited as long as a command of the user may be input. The electronic interface includes a display capable of touch input.

The output unit is used to transfer information to the user by expressing output of the autofluorescence image evaluation device 200 to the outside, and may include a display 222, a light emitting diode (LED), a speaker 223, and the like, for expressing visual output, auditory output, or tactile output.

The autofluorescence image evaluation device 200 may include a peripheral device interface unit for data transmission to various types of connected external devices, and may include a memory card port, an external device input/output (I/O) port, and the like.

The autofluorescence image evaluation device 200 includes a memory 240 storing the autofluorescence image received or captured by the camera and storing a code for driving a processor 230.

The autofluorescence image evaluation device 200 analyzes the autofluorescence image loaded by the processor 230 and determines information related to an opacity degree or a cataract grade of the examined eye. The autofluorescence image analyzed by the autofluorescence image evaluation device 200 is an image generated based on the output of the image sensor on which the autofluorescence of the examined eye generated by the illumination of the light source passes through the filter disposed between the image sensor and the examined eye and including the plurality of parts having the different light transmittances and is then incident.

An evaluation method of the autofluorescence image evaluation device 200 according to an embodiment of the present disclosure will be described with reference to FIG. 8.

The autofluorescence image evaluation device 200 receives the autofluorescence image captured by the analysis filter or at least a portion of the autofluorescence image from the autofluorescence imaging device, the control computing device of the autofluorescence imaging device, or the CIS/PACS/HIS server device (S310). The autofluorescence image evaluation device 200 may receive only portions of the autofluorescence image corresponding to the first part and/or the second part of the analysis filter including the plurality of parts having the different light transmittances of the autofluorescence image.

The autofluorescence image evaluation device 200 may analyze gray levels of portions of the autofluorescence image corresponding to the second part and/or the first part and the second part of the analysis filter (S320), and determine the information related to the opacity degree or the cataract grade of the examined eye (S330).

When the autofluorescence image evaluation device 200 receives the entirety of the autofluorescence image captured based on the analysis filter, the autofluorescence image evaluation device 200 may detect a portion of the autofluorescence image corresponding to the second part of the analysis filter based on a machine learning-based learning model. In this case, the learning model may be a learning model learned from a figure pattern of the second part of the analysis filter or learned from an image labeling a portion corresponding to the second part of the analysis filter in the autofluorescence image.

When the autofluorescence image evaluation device 200 receives the entirety or a portion of the autofluorescence image captured based on the analysis filter, the autofluorescence image evaluation device 200 may input a portion of the autofluorescence image corresponding to the second part of the analysis filter to a machine learning-based learning mode based on the machine learning-based learning model and determine a degree of opacity of the crystalline lens, a degree of progress of cataract, or a degree of progress of presbyopia (S330). In this case, the learning model may be a learning model learned from an image in which the entirety of the autofluorescence image or the portion of the autofluorescence image corresponding to the second part of the analysis filter is labeled according to the degree of opacity of the crystalline lens, the degree of progress of cataract, or the degree of progress of presbyopia.

The machine learning-based learning model may include a neural network having a convolutional neural network (CNN), a region-based CNN (R-CNN), a convolutional recursive neural network (C-RNN), a fast R-CNN, a faster R-CNN, and a region-based fully convolutional Network (R-FCN), a you only look once (YOLO), or a single shot multibox detector (SSD) structure.

The learning model may be implemented in hardware, software, or a combination of hardware and software, and when a portion or the entirety of the learning model is implemented in software, one or more instructions constituting the learning model may be stored in the memory.

In an embodiment, the autofluorescence image evaluation device 200 may determine the information related to the opacity degree or the cataract grade of the examined eye based on a comparison result between the gray levels of the portions of the autofluorescence image corresponding to the first part and the second part of the analysis filter with each other.

In an embodiment, referring to FIG. 9, images 910, 920, 930, and 940, captured in color of, examined eyes having opacity grades of 2, 3, 4, and 6, respectively, according to a lens opacities classification system (LOCS) standard, and images 911, 921, 931, and 941 captured based on the analysis filter (FIGS. 6C and 11) which is an embodiment of the present disclosure, may be confirmed. The autofluorescence image evaluation device 200 may receive the images 911, 921, 931, and 941 captured based on the analysis filter (FIGS. 6C and 11), determine differences between gray levels of portions 913, 923, 933, and 943 of the autofluorescence images corresponding to the first parts and gray levels of portions 913, 923, 933, and 943 of the autofluorescence images corresponding to the second parts, and determine the information related to the opacity degree or the cataract grade of the examined eye based on the differences.

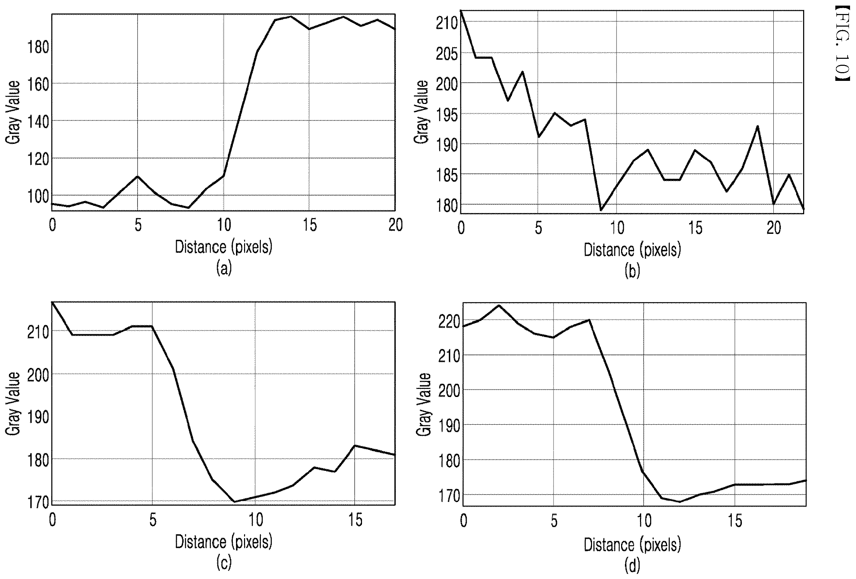

FIG. 10 is a diagram illustrating gray level changes between the portions 913, 923, 933, and 943 of the autofluorescence images corresponding to the second parts and the portions 913, 923, 933, and 943 of the autofluorescence images corresponding to the first parts.

Referring to FIG. 10, it may be confirmed that as an opacity degree of the examined eyes based on LOCS becomes higher, brightness due to autofluorescence increases in the portions 913, 923, 933, and 943 of the autofluorescence images corresponding to the second part.

In an embodiment, the autofluorescence image evaluation device 200 may recognize an optic nerve or a blood vessel in the autofluorescence image by the machine learning-based learning model, and analyze gray levels of a portion of the autofluorescence image corresponding to the first part and a portion of the autofluorescence image corresponding to the second part that do not overlap the optic nerve or blood vessel part. In this case, the learning model may be a model learned from an image in which the optic nerve or the blood vessel is labeled in the autofluorescence image.

In another embodiment, the autofluorescence image evaluation device 200 may analyze gray levels of pixels that do not overlap the optic nerve or the blood vessel part in the portion of the autofluorescence image corresponding to the first part and the portion of the autofluorescence image corresponding to the second part.

In another embodiment, the autofluorescence image evaluation device 200 may analyze gray levels of a plurality of portions of the autofluorescence image corresponding to the second part. For example, the autofluorescence image evaluation device 200 may quantitatively evaluate a degree of cataract based on a difference between gray scale values in image portions corresponding to two figures corresponding to the second part of the analysis filter in the autofluorescence image. That is, the autofluorescence image evaluation device 200 may analyze a gray level difference between a part of the autofluorescence image corresponding to a figure positioned at the center of the filter portion of the analysis filter and a part of the autofluorescence image corresponding to a figure positioned at an outer side of the filter portion.

The present invention described above can be embodied as a computer readable code on a medium in which a program is recorded. A computer readable medium may include all kinds of recording devices in which data that may be read by a computer system are stored. An example of the computer readable medium may include a hard disk drive (HDD), a solid state disk (SSD), a silicon disk drive (SDD), a read only memory (ROM), a random access memory (RAM), a compact disk read only memory (CD-ROM), a magnetic tape, a floppy disk, an optical data storage, and the like. In addition, the computer may also include a processor of each device.

Meanwhile, the program may be specially designed and configured for this disclosure, or may be known and available to those skilled in the art of computer software. Examples of the program include a high-level language code capable of being executed by a computer using an interpreter, or the like, as well as a machine language code made by a compiler.

In the specification of the present disclosure (particularly in the claims), the use of the term "above" and similar indicating terms may correspond to both singular and plural. In addition, when the range is described in the present disclosure, as including the invention to which individual values belonging to the range are applied (unless otherwise stated), each individual value constituting the range is described in the detailed description of the invention.

Unless an order is explicitly stated or stated to the contrary for steps constituting the method according to the present disclosure, the steps may be performed in any suitable order. The present disclosure is not necessarily limited to the order of description of the steps. The use of all examples or exemplary terms (e.g., etc.) in this disclosure is simply to explain the present disclosure in detail, and the range of the present disclosure is limited due to the examples or exemplary terms unless limited by the claims. In addition, those skilled in the art can appreciate that various modifications, combinations and changes can be made according to design conditions and factors within the scope of the appended claims or equivalents thereof.

Therefore, the spirit of the present disclosure should not be limited to these exemplary embodiments, but the claims and all of modifications equal or equivalent to the claims are intended to fall within the scope and spirit of the present disclosure.

The present disclosure is a result of research conducted with support from Korea University.
Research support: Korea University
Project number: K2107901
Project name: Development of holography-based optical technology for alleviating visual dysfunction due to cataracts and corneal opacity

## Claims

1. An autofluorescence imaging device comprising:
- a light source illuminating an examined eye through a preset optical path;
- an image sensor imaging the examined eye of which at least a portion emits light as autofluorescence by the illumination of the light source; and
- a filter disposed between the image sensor and the examined eye,
wherein the filter includes a plurality of portions each having different light transmittances.

2. The autofluorescence imaging device of claim 1, wherein the image sensor is set so that the autofluorescence of the examined eye generated by the illumination of the light source passes through the filter and is then incident thereon.

3. The autofluorescence imaging device of claim 1, wherein the filter includes a first part and a second part where a plurality of figures having lower light transmittance than the first part are formed.

4. The autofluorescence imaging device of claim 3, wherein the second part includes a plurality of figures disposed to be spaced apart from each other, and the plurality of figures have a form in which they are vertically symmetrical or horizontally symmetrical to each other.

5. The autofluorescence imaging device of claim 3, wherein the second part includes a plurality of ring-shaped figures and a plurality of circle-shaped figures of which inner portions have uniform light transmittance.

6. The autofluorescence imaging device of claim 3, wherein the second part includes a plurality of quadrangle-shaped figures of which inner portions have uniform light transmittance.

7. The autofluorescence imaging device of claim 6, wherein at least two of the plurality of quadrangle-shaped figures have different areas.

8. The autofluorescence imaging device of claim 7, wherein in at least some of the plurality of quadrangle-shaped figures, a figure close to a peripheral portion of the filter has a greater area than a figure close to a central portion of the filter.

9. The autofluorescence imaging device of claim 6, wherein at least two of the plurality of quadrangle-shaped figures have different aspect ratios.

10. An autofluorescence image evaluation device comprising:
- a processor; and
- a memory electrically connected to the processor and storing at least one code executed by the processor,
wherein the memory stores a code causing the processor to analyze an autofluorescence image of an examined eye and determine information related to an opacity degree or a cataract grade of the examined eye, and
the autofluorescence image is an image generated based on output of an image sensor on which autofluorescence of the examined eye generated by illumination of a light source passes through a filter disposed between the image sensor and the examined eye and including a plurality of portions each having different light transmittances and is then incident.

11. The autofluorescence image evaluation device of claim 10, wherein the memory further stores a code causing the processor to determine the information related to the opacity degree or the cataract grade based on a comparison result between gray levels of a plurality of image parts corresponding to the plurality of parts of the filter having the different light transmittances in the autofluorescence image.

12. The autofluorescence image evaluation device of claim 11, wherein the autofluorescence image includes a first image part and a second image part respectively corresponding to a first part of the filter and a second part where a plurality of figures having lower light transmittance than the first part are formed, and
the memory further stores a code causing the processor to determine the information related to the opacity degree or the cataract grade based on a comparison result between gray levels of the first image part and the second image part.

13. The autofluorescence image evaluation device of claim 12, wherein the memory further stores a code causing the processor to recognize an optic nerve or a blood vessel in the autofluorescence image and determine the information related to the opacity degree or the cataract grade based on the comparison result between the gray levels of the first image part and the second image part where the optic nerve or the blood vessel does not exist or determine the information related to the opacity degree or the cataract grade based on a comparison result between gray levels of parts of the first image part and the second image part where the optic nerve or the blood vessel does not exist.

14. The autofluorescence image evaluation device of claim 12, wherein the memory further stores a code causing the processor to determine the information related to the opacity degree or the cataract grade based on a difference between the gray levels of the first image part and the second image part.

15. An operation method of an autofluorescence imaging device, comprising:
- emitting light from a light source so as to illuminate an examined eye through a preset optical path, by a processor; and
- imaging the examined eye of which at least a portion emits light as autofluorescence by the illumination of the light source by controlling an image sensor, by the processor,
wherein the imaging of the examined eye includes generating an output signal by the image sensor based on the autofluorescence of the examined eye passing through a filter including a plurality of portions each having different light transmittances and then incident on the image sensor.

16. The operation method of an autofluorescence imaging device of claim 15, further comprising:
- confirming a position of the filter by the processor;
- confirming an imaging mode by the processor; and
- outputting a message by the processor when the position of the filter and the imaging mode do not coincide with each other.

17. The operation method of an autofluorescence imaging device of claim 15, further comprising:
- generating a fundus autofluorescence image based on the output signal of the image sensor by the processor; and
- adding image type information indicating imaging based on the filter to the fundus autofluorescence image by the processor.

18. An evaluation method of an autofluorescence image evaluation device, comprising:
- receiving at least a portion of an autofluorescence image of an examined eye by a processor; and
- analyzing the autofluorescence image and determining information related to an opacity degree or a cataract grade of the examined eye, by the processor,
wherein the autofluorescence image is an image generated based on output of an image sensor on which autofluorescence of the examined eye generated by illumination of a light source passes through a filter disposed between the image sensor and the examined eye and including a plurality of portions each having different light transmittances and is then incident.

19. The evaluation method of an autofluorescence image evaluation device of claim 18, wherein the determining of the information includes determining the information related to the opacity degree or the cataract grade based on a comparison result between gray levels of a plurality of image parts corresponding to the plurality of parts of the filter having the different light transmittances in the autofluorescence image.
